Europäisches Patentamt

European Patent Office

Office européen des brevets

(19)

(11) Veröffentlichungsnummer : **0 517 126 A1**

(12) ## EUROPÄISCHE PATENTANMELDUNG

(21) Anmeldenummer : 92109170.8

(51) Int. Cl.$^5$ : **A61K 37/02**

(22) Anmeldetag : 01.06.92

(30) Priorität : 31.05.91 DE 4118348

(43) Veröffentlichungstag der Anmeldung :
09.12.92 Patentblatt 92/50

(84) Benannte Vertragsstaaten :
AT BE CH DE DK ES FR GB GR IT LI LU MC NL
PT SE

(71) Anmelder : WOGEPHARM GmbH
Afrastrasse 21
W-5030 Hürth (DE)

(72) Erfinder : Diezel, Wolfgang, Prof. Dr. sc. med.
Anton-Saefkow-Platz 3
O-1156 Berlin (DE)

(74) Vertreter : Werner, Hans-Karsten, Dr. et al
Deichmannhaus am Hauptbahnhof
W-5000 Köln 1 (DE)

(54) **Verwendung von Diacetyl-Splenopentin bei der Chemo- und Strahlentherapie.**

(57) Es hat sich gezeigt, daß DA-SP5 direkt die Proliferation unreifer Stammzellen des Knochenmarks beschleunigen kann bzw. die Wirkung des Kolonien-stimulierenden Faktors GM-CSF synergistiche erhöht. Es wurde jetzt festgestellt, daß als Folge dieser Wirkung die zytotoxische Schädigung von Knochenmarkstammzellen (und damit die verminderte Entstehung immunologiser Abwehrzellen) durch Chemotherapeutika bzw. infolge Bestrahlung der Zellen, kompensiert werden kann.

EP 0 517 126 A1

Jouve, 18, rue Saint-Denis, 75001 PARIS

Die Erfindung betrifft die therapeutische Anwendung von Diacetyl-Splenopentin (DA-SP5:(N$^\alpha$-acetyl-L-arginyl)-(N$^\varepsilon$-acetyl-L-lysyl)-L-glutamyl-L-valyl-L-tyrosin)) bei Krebserkrankungen des Menschen zusammen mit Zytostatika oder nach einer Bestrahlungstherapie bzw. vor jeder operativen Entfernung von Krebsgeschwülsten.

Eine chemotherapeutische Therapie bzw. eine Bestrahlungstherapie ist bei bestimmten Krebserkrankungen notwendig. Dadurch werden aber auch Stammzellen des Knochenmarks geschädigt, und es resultiert eine Abnahme der Anzahl immunologischer Abwehrzellen im Blut, da sich diese aus Stammzellen bilden. Als Folge muß oftmals die Krebstherapie abgebrochen werden.

DA-SP5 erhöht die Proliferation und die Differenzierung der Knochenmarkstammzellen - die o. g. Wirkung der Chemotherapeutika bzw, einer Bestrahlungstherapie auf diese Zellen kann somit kompensiert werden.

Das Anwendungsgebiet der Erfindung ist die pharmazeutische Industrie.

Entsprechend einer Hypothese von BERENBLUB (Cancer 47 (1981) Seite 2346) entstehen im menschlichen organismus permanent Krebszellen ("Initiation" des Krebsgeschehens). Diese Zellen werden jedoch auch permanent durch Zellen des Immunsystems zerstört. Erst wenn Krebszellen überleben, resultiert Krebs (Phase der "Promotion" des Krebsgeschehens). Das Überleben von Krebszellen kann vor allem durch eine temporäre Immunosuppression begünstigt werden. Dies schließt die Immunosupression bei jeder Operation in Vollnarkose ein. - Ist Krebs ausgebrochen, gilt es folgende Therapieprinzipien zu verfolgen: Die radikale Entfernung aller Tumorzellen durch operative Maßnahmen bzw. durch eine Bestrahlungstherapie und die Zerstörung möglichst aller Tumorzellen durch eine nachfolgende zytotoxische Chemotherapie. Die zytotoxische Chemotherapie schädigt auch die Stammzellen des Knochenmarks - es resultiert eine Immunosuppression und eine verminderte immunobiologische Krebsabwehr. Daher sollte sich jeder Krebsoperation bzw. jeder Chemotherapie eine Therapie mit "Immunmodulatoren" anschließen (H.WRBA: Kombinierte Tumortherapie, Hippokrates Verlag, Stuttgart, 1990, Seite 146 -184). Derzeit werden dafür therapeutisch angewandt: Thymusfaktoren (Thymushormone), Interferone, Interleukin-2. Diese Substanzen erhöhen primär die Funktion schon vorhandener Immunzellen. Sie erhöhen die Nachbildung neuer immunologischer Abwehrzellen nicht. Dies soll durch die Substanz DA-SP5 nach Abnahme der immunologischen Abwehrzellen Infolge Krebschemotherapie bzw. infolge Bestrahlungstherapie erreicht werden.

Der im Anspruch 1 angegebenen Erfindung liegt das Problem zugrunde, der medizinischen Praxis ein therapeutisches Mittel in die Hand zu geben, welches bei Krebspatienten die Bildung immunologischer Abwehrzellen nach Abnahme dieser Zellen infolge zytotoxischer Chemotherapie bzw. infolge Bestrahlungstherapie beschleunigt.

Es wurde gefunden, daß als Folge einer Therapie mit Zytostatika bzw. durch eine Bestrahlungstherapie sich im Blut der entsprechend behandelten Patienten die Zahl immunologischer Abwehrzellen vermindert. Dies resultiert, weil durch die o. g. Therapie auch die Stammzellen des Knochenmarks (aus diesen entstehen die immunologischen Abwehrzellen) zytotoxisch geschädigt werden. Die Substanz DA-SP5 erhöht die Nachbildung von immunologischen Abwehrzellen aus Stammzellen des Knochenmarks (W. DIEZEL et al. (1989): DD-PS 268160 Al). Es hat sich gezeigt, daß DA-SP5 direkt die Proliferation unreifer Stammzellen des Knochenmarks beschleunigen kann bzw. die Wirkung des Kolonien-stimulierrenden Faktors GM-CSF synergistisch erhöht. Überraschenderweise wurde jetzt festgestellt, daß als Folge dieser Wirkung die zytotoxische Schädigung von Knochenmarkstammzellen (und damit die verminderte Entstehung immunologischer Abwehrzellen) durch Chemotherapeutika bzw. infolge Bestrahlung der Zellen, kompensiert werden kann.

Die erfindungsgemäße Wirkung wurde dadurch festgestellt, indem BALB/c-Mäuse hochdosiert mit zytotoxischen (Zellproliferations-hemmenden) Therapeutika systemisch behandelt wurden. Anschließend erfolgte eine Therapie mit DA-SP5 bzw. es wurde lediglich 0,9%ige NaCl-Lösung (Kontrallgruppe) appliziert. Während dieser Therapie wurde untersucht, ob die DE-SP5-behandelten Tiere zeitlich eher befähigt wurden "Knochenmarkstammzell-Kolonien" entsprechend der Norm zu bilden. Eine diesbezüglich schnellere Bildung korreliert mit einer schnelleren Entstehung entsprechender immunologischer Abwehrzellen im Blut. In einer weiteren Versuchsserie wurden BALB/c-Mäuse subletal bestrahlt, und anschließend ebenfalls mit oder ohne DA-SP5 behandelt. Auch bei diesen Tieren wurde untersucht, ob sie zeitlich eher befähigt waren, "Knochenmarkzell-Kolonien" entsprechend der Norm zu bilden.

Das erfindungsgemäß verwendete Mittel wurde in Mengen von 0,05mg bis 1,0 mg pro kg Körpergewicht verabreicht.

Die nun folgenden Ausführungsbeispiele erläutern die Erfindung:

Beispiel 1:

Chemotherapeutische Behandlung von BALB/c-Mäusen mit Dexamethason und nachfolgende systemische Behandlung der Tiere mit oder ohne DA-SP5 (Tabelle 1).

2

6 bis 8 Wochen alten BALB/c-Mäusen wurden an den Tagen +1 bis +5 je 150 mg pro 1 kg Körpergewicht Dexamethason intraperitoneal appliziert. Anschließend wurde eine Tiergruppe mit DA-SP5 folgendermaßen behandelt: Intraperitoneale Applikation von 10 µg DA-SP5, gelöst in 0,9%iger NaCl-Lösung (pH 7,3), dreimal wöchentlich (Montag/Mittwoch/Freitag). Eine zweite Tiergruppe wurde lediglich mit 0,9%iger NaCl-Lösung therapiert. In differenten Zeitabständen nach der Dexamethason-Therapie (Tabelle 1) wurden die Knochenmarkstammzellen der Tiere hinsichtlich ihrer Fähigkeit untersucht, "Zell-Kolonien" zu bilden. Der Agar-Kolonientest (CFU-Test) wurde entsprechend der von D. METCALF angegebenen Methodik durchgeführt (D. METCALF: Hemopoitic colonies, Springer Verlag, Berlin-Heidelberg-New York, 1977, Seite 223-227). Als Quelle der "Kolonien-Stimulierenden" Aktivität wurde 2%iges lungenkonditioniertes Medium eingesetzt. (Methodik: M. MACIEJEWSKI et al.: European Journal of Immunology 20 (1990) Seite 1209-1213).

Tabelle 1.

"Kolonien"-bildende Zellen (CFU) pro Femur.

Chemotherapeutische Behandlung von BALB/c-Mäusen mit Dexamethason und nachfolgende Behandlung der Tiere mit oder ohne DA-SP5.

| Tiergruppe[1] | Kolonien pro Femur $(\bar{x} \pm s)$[2] | | | | | |
|---|---|---|---|---|---|---|
| | DA-SP5 Therapie | | | | | |
| | Tag Nr. 10 | <p | Tag Nr. 20 | <p | Tag Nr. 40 | < p |
| Dexamethason ohne DA-SP5 | 6 ± 6 | | 32 ± 11 | | 61 ± 25 | |
| Dexamethason + DA-SP5 | 10 ± 8 | n.s. | 55 ± 21 | n.s. | 126 ± 18 | 0,001 |

[1] Jede Tiergruppe bestand aus 5 Tieren

[2] "Kolonien"-Normalwert: 121 ± 23 (n = 10)

[3] Statistik: Mann-Whitney-Test

[4] n.s. = nicht signifikant

Beispiel 2:

Chemotherapeutische Behandlung von BALB/c-Mäusen mit methotrexat und nachfolgender systemischer Behandlung der Tiere mit oder ohne DA-SP5 (Tabelle 2).

Behandlung der Tiere an den Tagen +1 bis +5 mit 175 mg Methotrexat pro 1 kg Körpergewicht. Gleichartige weitere Versuchsdurchführung und DA-SP5 Medikation wie im Ausführungsbeispiel Nr. 1 angegeben.

Tabelle 2.

"Kolonien"-bildende Zellen (CFU) pro Femur.

Chemotherapeutische Behandlung von BALB/c-Mäusen mit Methotrexat und nachfolgende Behandlung der Tiere mit oder ohne DA-SP5.

| Tiergruppe[1] | Kolonien pro Femur $(\bar{x} \pm s)$[2] | | |
|---|---|---|---|
| | DA-SP5 Therapie | | |
| | Tag Nr. 10   <p[3] | Tag Nr. 20   <p | Tag Nr. 40 < p |
| Methotrexat ohne DA-SP5 | $6 \pm 4$ | $24 \pm 12$ | $65 \pm 12$ |
| Methotrexat + DA-SP5 | $9 \pm 6$   n.s.[4] | $53 \pm 24$ — 0,05 | $110 \pm 20$   0,001 |

[1] Jede Tiergruppe bestand aus 5 Tieren

[2] "Kolonien"-Normalwert: $121 \pm 23$ (n = 10)

[3] Statistik: Mann-Whitney-Test

[4] n.s. = nicht signifikant

Beispiel 3:

Subletale Röntgenbestrahlung von BALB/c-Mäusen und nachfolgende systemische Behandlung der Tiere mit oder ohne DA-SP5 (Tabelle 3).

Einmalige subletale Bestrahlung der BALB/c-Mäuse mit 550 Gy. Gleichartige weitere Versuchsdurchführung und DA-SP5 Medikation wie im Ausführungsbeispiel Nr. 1 angegeben.

Tabelle 3.

"Kolonien"-bildende Zellen (CFU) pro Femur.

Einmalige Röntgenbestrahlung von BALB/c-Mäusen mit 550 Gy und

nachfolgende Behandlung der Tiere mit oder ohne DA-SP5.

| Tiergruppe[1] | Kolonien pro Femur $(\bar{x} \pm s)$[2] | | |
|---|---|---|---|
| | DA-SP5 Therapie | | |
| | Tag Nr.10 <p[3] | Tag Nr.20 < p | Tag Nr.40 < p |
| Röntgenbestrahlung ohne DA-SP5 | 6 ± 4 | 58 ± 12 | 98 ± 8 |
| Röntgenbestrahlung + DA-SP5 | 8 ± 6   n.s.[4] | 116 ± 10   0,001 | 126 ± 12   0,05 |

[1] Jede Tiergruppe bestand aus 5 Tieren

[2] "Kolonien"-Normalwert: 121 ± 23 (n = 10)

[3] Statistik: Mann-Whitney-Test

[4] n.s. = nicht signifikant

**Patentansprüche**

1. Substanz der Zusammensetzung N$\alpha$-acetyl-L-arginyl)-(N$\varepsilon$-acetyl-L-Lysyl)-L-glutamyl-L-valyl-L-tyrosin) zur Verwendung als Arzneimittel, dadurch gekennzeichnet, daß die Substanz bei Krebspatienten vor jeder Operation und nach jeder chemotherapeutischen Behandlung und bei jeder Bestrahlungstherapie in Mengen von 0,05 mg bis 1,0 mg pro 1 kg Körpergewicht therapeutisch eingesetzt wird.

2. Substanz nach Anspruch 1, dadurch gekennzeichnet, daß die Substanz intramuskulär bzw, subkutan appliziert wird.

Europäisches
Patentamt

**EUROPÄISCHER RECHERCHENBERICHT**

Nummer der Anmeldung

EP 92 10 9170

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 307 553 (VEB BERLIN-CHEMIE) <br> * Seiten 5,6; Beispiele 4,14,15, Tabelle 3.1,5.1; Ansprüche * <br> --- | 1-2 | A 61 K 37/02 |
| X | ARCHIV FÜR GESCHWULSTFORSCHUNG, Band 59, Nr. 3, 1989, Seiten 161-164; W. DIEZEL et al.: "Splenopentin - influence on antibody formation in immunosuppressed animals and on phagocytic capability of human granulocytes" <br> * Ganzes Dokument * <br> --- | 1-2 | |
| X | INT. J. IMMUNOPHARMACOL., Band 12, Nr. 7, 1990, Seiten 761-768, Pergamon Press plc., GB; H.A. WEBER et al.: "Splenopentin (DAc-SP-5) accelerates the restoration of myelopoietic and immune systems after sublethal radiation in mice" <br> * Ganzes Dokument * <br> --- | 1-2 | |
| X | EXP. CLIN. ENDOCRINOL., Band 94, Nr. 1/2, 1989, Seiten 223-225; R. ECKERT et al.: "Splenopentin-induced reconstitution of the immune response after total body irradiation: optimization of treatment regime" <br> * Ganzes Dokument * <br> ----- | 1-2 | **RECHERCHIERTE SACHGEBIETE (Int. Cl.5)** <br><br> A 61 K |

Der vorliegende Recherchenbericht wurde für alle Patentansprüche erstellt

| Recherchenort | Abschlußdatum der Recherche | Prüfer |
|---|---|---|
| DEN HAAG | 20-08-1992 | GOETZ G. |